# EUROPÄISCHE PATENTSCHRIFT

(11) **EP 0 913 687 B1**
(45) Veröffentlichungstag und Bekanntmachung des Hinweises auf die Patenterteilung: **29.12.2004**
(21) Anmeldenummer: 98119670.2
(22) Anmeldetag: 17.10.1998
(51) Int. Cl.: G01N 27/48, G01N 33/22

(54) **Verfahren und Vorrichtung zur Detektion schwerflüchtiger Substanzen**
Method and device for detecting low volatile substances
Procédé et dispositif pour la détection de substances peu volatiles

(30) Priorität: 31.10.1997 DE 19748124
(43) Veröffentlichungstag der Anmeldung: 06.05.1999
(73) Patentinhaber: Fraunhofer-Gesellschaft zur Förderung der angewandten Forschung e.V., 80686 München (DE)
(72) Erfinder: Krausa, Michael Dr., 76327 Pfinztal (DE); Schorb, Klaus Dipl.-Ing., 76287 Rheinstetten (DE); Krebs, Stefan Dipl.-Ing., 76229 Karlsruhe (DE); Becker, Frank, 76227 Karlsruhe (DE)

(56) Entgegenhaltungen:
- EP-A- 0 531 745
- EP-A- 0 665 431
- DE-A- 2 904 886
- DE-A- 4 109 909
- DE-A- 4 223 228
- US-A- 4 146 437
- US-A- 5 518 590
- FINE D A ET AL: "VOLTAMETRIC ANALYSIS OF ORDNANCE MATERIALS PART 1. DETECTION AND QUANTITATION OF NITRATE ESTERS AND VARIOUS NITRO COMPOUNDS IN WATER BY VOLTAMMETRY" REPORT NWC-TP-6505, Bd. 6505, 1. April 1984, Seiten 3-25, XP002013655

## Beschreibung

Die Erfindung betrifft ein Verfahren und eine Vorrichtung zur Detektion schwerflüchtiger Substanzen nach dem Oberbegriff des Anspruchs 1 bzw. des Anspruchs 8.

Um beispielsweise gasförmige, schwerflüchtige Substanzen nachzuweisen, werden zumeist potentiostatische (amperometrische) Messsysteme verwendet, bei denen an einer zumeist porösen, großen Elektrode ein Potential vorgegeben wird. Infolge der elektrochemischen Oxidation oder Reduktion erfolgt dann der quantitative Nachweis der vorhandenen Substanzen über die Größe des fließenden Stroms. Der qualitative Nachweis erfolgt dabei vorzugsweise mittels der Gaschromatographie oder der Hochdruck-Flüssigkeitschromatographie (HPCL) oder anderer Methoden, bei denen die genannten elektrochemischen Systeme als Detektoren eingesetzt werden.

Bei den herkömmlichen elektrochemischen Sensorsystemen zur Gasphasenanalytik, bei denen die Systeme auch auf Halbleiterbasis beruhen können, sind die Elektroden durch eine Membran oder Polymerschicht vom Gasraum abgetrennt, wobei dann die nachzuweisende Substanz durch eine durchlässige Membran hindurch diffundiert und anschließend in mit den Elektroden in Kontakt stehenden Innenelektrolyten gelöst wird. Der Nachweis der jeweiligen Substanz erfolgt dann durch elektrochemische Oxidation bzw. Reduktion an der Arbeitselektrode. Hierbei wird, wie bereits gesagt, ein festes Potential an der Arbeitselektrode. Hierbei wird, wie bereits gesagt, ein festen Potential an der Arbeitselektrode vorgegeben.

Nachteilig bei derartigen Verfahren, wie der Gaschromatographie, ist, dass sie relativ zeitaufwendig und teuer sind. Werden Böden oder Flüssigkeiten untersucht, so müssen zunächst Proben genommen und an einen dafür vorgesehenen Analyseort gebracht werden.

Des weiteren werden die meisten Sprengladungen wegen der technologisch einfachen Herstellung und Verarbeitbarkeit aus Trinitrotoluol (TNT) oder mit Zusatz von TNT hergestellt. Da derartiger Sprengstoff auch bei erpresserischen oder terroristischen Drohungen sowie Anschlägen Verwendung findet, stellt die Auffindung derartiger Sprengstoffe, z.B. bei der Gepäck- und Passagierkontrolle im Flugverkehr, eine wichtige Aufgabe dar, um eine Gefährdung von Menschenleben zu verhindern. Auch bei der Minensuche von kunststoffummantelten Minen ist eine möglichst schnelle und unkomplizierte Detektion derartiger Minen unerlässlich. Bei dem bei der Sprengstoff- bzw. Minenherstellung verwendeten TNT handelt es sich um einen Feststoff, der in kleinen Mengen Dampf an die Umgebung abgibt.

Um derartige Kontaminationen in Böden, Flüssigkeiten und Gasen detektieren zu können, wurde bereits in der EP-A-0 665 431 eine Vorrichtung vorgeschlagen, die auf der Grundlage der zyklischen Voltammetrie arbeitet. Diese Vorrichtung weist ein Sensorelement mit drei Elektroden, nämlich einer Arbeitselektrode, einer Bezugselektrode sowie einer Gegenelektrode auf, die mit einer regelbaren Spannungsquelle sind. An die Arbeitselektrode wird eine Spannung angelegt, durch deren zyklische Verringerung und Erhöhung sowie gleichzeitige Messung der Stromstärke in Abhängigkeit von der eingestellten Spannung sogenannte Voltammogramme in Form von Strom-Spannungs-Kurven erhalten werden. Die Spannungswerte bei jeweils an der Arbeitselektrode auftretenden kathodischen und anodischen Stromspitzen für Reduktion und Oxidation der Redoxpaare stellen ein qualitatives Maß für die enthaltenen Kontaminationen dar. Aus dem Quotienten der Stromstärke des anodischen Strommaximums und des gemessenen Stromes im sogenannten Doppelschichtbereich wird dann der TNT-Gehalt bestimmt. Mittels der genannten Vorrichtung kann zwar der TNT-Gehalt oder derjenige anderer Substanzen an sich bestimmt werden, jedoch lässt die Nachweisempfindlichkeit insbesondere bei sehr kleinen Dampfdrücken doch zu wünschen übrig. Insbesondere eine Bestimmung von Substanzen in der Gasphase ist mit dieser Vorrichtung nicht möglich, da zur Bestimmung gasförmiger Substanzen eine Membran verwendet werden muss, durch die eine Durchflusszelle von der Elektrolylösung getrennt wird.

Es handelt sich hier um die Bestimmung der Konzentration eines bekannten Stoffes hinsichtlich seiner Konzentration. Darüber hinaus setzt der Gegenstand der Druckschrift eine Kalibrierung der Elektrodenanordnung voraus, wobei diese Kalibrierung notwendigerweise bei einer bekannten, insbesondere hinsichtlich ihrer Konzentration bekannten Lösung zu erfolgen hat, die sich demgemäß von den späteren Lösungen, deren Konzentration an Persäure bestimmt werden soll, unterscheidet. Bei jeder der beiden Lösungen - Kalibrierungslösung und in ihrer Konzentration unbekannten Lösung wird das Voltammogramm lediglich einmal durchlaufen.

Die US 5,518,590 betrifft einen elektrochemischen Sensor zur Prüfung der Zersetzung von Motorölen, insbesondere hinsichtlich der Antioxidationseigenschaften durch Bestimmung des Anteils von Antioxidationsmitteln im Öl. Der Sensor hat zwei oder drei Elektroden, nämlich eine Arbeits- und eine Gegenelektrode sowie gegebenenfalls eine Bezugselektrode. Auch hier werden vorgegebene feste elektroaktive Zusätze hinsichtlich ihrer Konzentration gemessen, nicht aber zunächst aus einer Vielzahl von möglichen Schadenstoffen zunächst die jeweilig vorliegenden qualitativ bestimmt und diese dann quantitativ hinsichtlich ihrer Konzentration gemessen. Die Elektroden sind durch eine Membran von der zu untersuchenden Substanzen getrennt, die das Hindurchtreten derselben erschwert, z.T. diese absorbiert, so dass hierdurch die Messempfindlichkeit zumindestens wesentlich beeinträchtigt wird.

Das gleiche gilt grundsätzlich für den Gegenstand der DE 29 04 886 A1. Diese Druckschrift zeigt eine kompliziert aufgebaute Zelle zur polarographischen Analyse eines in einer Probe enthaltenen Bestandteils mit einem ersten Gehäuseteil, das einen Trägerkörper für Elektroden umgebenden Raum zur Aufnahme eines Elektrolyten sowie einer den Trägerkörper umgebenden ersten Elektrode aufweist. Eine zweite Elektrode durchsetzt den Trägerkörper zentrisch und mündet in einer gewölbten Endfläche desselben, die von einer für den zu untersuchenden Bestandteil durchlässigen, für einen Elektrolyten undurchlässigen Membran abgedeckt ist. Ein zweites mit dem ersten Gehäuseteil verbindbares Gehäuseteil ist zur dichtenden Halterung der Membran zwischen den beiden Gehäuseteilen und zur Zufuhr der Probe zu der der Endfläche abgewandten Seite der Membran ausgebildet. Weiterhin ist der außerhalb des an der Endfläche des Trägerkörpers anliegenden Anlagebereichs der Membran befindliche Teil derselben an der der Endfläche abgewandten Seite gegen Berührung mit der Probe abgedichtet. Auch hier wird das Hindurchtreten der zu untersuchenden Substanz durch die Membran selbst erschwert, wobei gerade auch die zu untersuchenden Substanzen durch eine solche Membran absorbiert werden, so dass eine derartige Vorrichtung zur Untersuchung der hier zu untersuchenden Substanzen nicht einsetzbar ist.

Der Erfindung liegt daher die Aufgabe zugrunde, ein Verfahren sowie eine Vorrichtung der eingangs genannten Art dahingehend weiterzubilden, dass elektrochemisch umsetzbare Substanzen auch bei sehr kleinen Konzentrationen bzw. Dampfdrücken qualitativ und quantitativ zuverlässig nachweisbar sind.

Erfindungsgemäß wird die genannte Aufgabe durch ein Verfahren der eingangs genannten Art dadurch gelöst, dass die kennzeichnenden Merkmale des Anspruchs 1 aufweist. Eine gattungsgemäße Vorrichtung sieht zur Lösung der Aufgabe weiterhin die kennzeichnenden Merkmale des Anspruchs 8 vor.

Es hat sich nämlich herausgestellt, daß das zyklische Voltammogramm sich bei mehrfachem Durchlaufen des Meßzyklus derart stabilisiert, daß sich infolge der Reduktion ein charakteristisches Maximum (kathodischer Strompeak) ausbildet. Durch Bestimmung der Differenzen der gemessenen Stromwerte kann das Auftreten eines solchen charakteristischen Maximums optimal und schnell ermittelt werden. Dadurch, daß erfindungsgemäß bei Auftreten eines kathodischen Strommaximums der zugehörige Spannungswert bestimmt, konstant gehalten und dann die Stromstärke potentiostatisch über die Zeit bis zur Sättigung bestimmt wird, wird so die Empfindlichkeit erhöht und die Nachweisdauer verkürzt. Der qualitative Nachweis erfolgt durch die Peakpotentialbestimmung und der quantitative Nachweis durch potentiostatische Messung der Stromhöhe. Hierdurch wird die Ansprechzeit deutlich verbessert. Es können beispielsweise Ansprechzeiten von weniger als 0,5 sec erreicht werden.

Bei der erfindungsgemäßen Vorrichtung ist vorgesehen, daß der benötigte Elektrolyt in einer dünnen Schicht auf die Stirnfläche des Sensorelementes aufgetragen ist und nicht wie beim Stand der Technik das Sensorelement in einen Elektrolyten eingetaucht wird. Durch die Dicke der Elektrolytschicht kann dann Einfluß auf die Nachweiszeit genommen werden. Mittels des erfindungsgemäßen Verfahrens sowie der erfindungsgemäßen Vorrichtung ist es so möglich, unterschiedlichste schwerflüchtige Substanzen auch in geringen Konzentrationen zuverlässig nachzuweisen. Dem optimal erreichbaren Potentialwert, bei dem ein kathodisches Strommaximum (negatives Vorzeichen) auftritt, nähert man sich durch das erfindungsgemäße Verfahren schrittweise.

Um die Meßzeit nicht unnötig zu erhöhen, wird bei einem Meßzyklus maximal der Potentialbereich zwischen Wasserstoff- und Sauerstoffentwicklung durchlaufen.

Die ermittelten bzw. bestimmten Werte bezüglich Stromhöhe, Differenzen und Potential können optisch oder aber akustisch ausgegeben werden. Beispielsweise können die akustischen Signale je nach ermittelter Konzentration der jeweiligen Substanz unterschiedlich sein. Dies ist insbesondere bei der Annäherung an eine Mine aufgrund der nun steigenden TNT-Konzentration vorteilhaft.

Ist das Meßmedium unter allen Bedingungen während des Durchlaufens der Meßzyklen gleich, so führt dies zu keiner Änderung des Potentials der Gegen- bzw. bei Verwendung einer solchen der Bezugselektrode. Entsprechend kann das beim kathodischen Strommaximum auftretende Potential zur Detektion der erfolgten TNT-Reduktion genutzt werden. Ändert sich jedoch das Meßmedium bzw. die Elektrolytzusammensetzung mit der Zeit, so führt dies zu Potentialverschiebungen. Aus diesem Grunde ist in Weiterbildung vorgesehen, nicht das absolute Potential am kathodischen Strommaximum zum Nachweis der Substanz zu nutzen, sondern die Potentialdifferenz der Spannungswerte/Potentiale zwischen einem eine Sauerstoffreduktion anzeigenden anodischen Strommaximum und dem kathodischen Strommaximum. Diese Potentialdifferenz zwischen kathodischem Strommaximum und Sauerstoffreduktionspeak bleibt für die jeweiligen Substanzen über mehrere Messungen konstant, wie sich gezeigt hat. Messungen haben ergeben, daß für 2,4-TNT und 2,6-TNT die Differenz ca. 840 mV beträgt. Es besteht lediglich eine Abhängigkeit vom jeweiligen Lösungsmittel und dem eingesetzten Elektrolyten. 3,4-TNT weist eine größere Differenz von 950 mV auf.

Für 2-Amino-5-Nitrotoluol wurden bei Messungen zwei Reduktionspeaks erhalten, von denen ein Peak eine Differenz zum Sauerstoffreduktionspeak von ca. 860 mV und ein zweiter eine Differenz von 480 mV aufweist. Die Kenntnis dieser Potentialdifferenzen vereinfacht so den Nachweis der jeweiligen Substanz.

Für kontinuierliche Messungen ist vorgesehen, daß eine zu untersuchende Substanz am Sensorelement mit der Arbeitselektrode vorbeigeführt wird. Bei einer gasförmigen Substanz wird diese vorzugsweise durch eine Durchflußzelle geleitet. Vorzugsweise wird die gasförmige Substanz dabei durch Pumpen durch die Durchflußzelle am Sensorelement vorbeigeführt. Auf diese Weise sind dann auch Substanzen geringer Konzentration und geringen Gleichgewichtsdampfdruckes nachweisbar.

Um den Elektrolyten einfach und schnell auf das Sensorelement auftragen zu können, ist dieser vorzugsweise dickflüssig oder aber gelartig ausgebildet. Ein derartiger Elektrolyt läßt sich nicht nur schnell auftragen, sondern haftet zuverlässig an der Stirnfläche des Sensorelementes. Auch kann ein solches Sensorelement einfach und schnell zur Bestimmung gasförmiger Substanzen in einen Gasraum eingebracht werden. Membranen werden nicht mehr benötigt, um Gasraum und Elektrolyt zu trennen.

Als Elektrolyten können neben anorganischen Verbindungen (Schwefelsäure, Perchlorsäure u.ä.) auch Gemische aus anorganischen und organischen Verbindungen (Aceton/Säure, Ethanol/Schwefelsäure, Hexan-1-Sulfonat/Schwefelsäure u.ä.) sowie rein organische Verbindungen (Aceton, Ethanol u.ä.) eingesetzt werden. Die Wahl des jeweiligen Elektrolyten hängt von der geforderten Sensitivität und Selektivität bei der Durchführung des erfindungsgemäßen Verfahrens ab und kann so speziell auf die nachzuweisende Substanz optimiert werden.

In bevorzugter Ausgestaltung ist vorgesehen, daß zumindest die Arbeits- und/oder Gegenelektrode Mikroelektroden sind. Vorzugsweise handelt es sich dabei dann sogar um Ultramikroelektroden. Hierdurch ist es möglich, die Messungen mit einer einfachen Zwei-Elektroden-Anordnung durchzuführen. Die Gegenelektrode dient gleichzeitig als Bezugselektrode. Dieser einfache Aufbau wird dadurch möglich, daß infolge der an den Mikroelektroden fließenden geringen Ströme das Potential der Gegenelektrode während eines Meßzyklus nahezu konstant bleibt.

Vorzugsweise ist die Arbeitselektrode im wesentlichen ringförmig von der Gegenelektrode umschlossen. Hierdurch wird bei kleinem Aufbau des Sensorelementes für eine große Fläche der Gegenelektrode gesorgt sowie für eine gleichmäßige Strombedeckung der Arbeitselektrode. Ferner ist die Gegenelektrode so im Elektrolyten im wesentlichen unpolarisierbar, da die mit dem Elektrolyten in Kontakt stehende Fläche der Gegenelektrode eben wesentlich größer als diejenige der Arbeitselektrode ist.

Dabei können die Sensorelemente auch mit mehreren Arbeitselektroden bestückt sein und/oder eine separate Bezugselektrode zusätzlich eingebaut sein.

In Weiterbildung ist vorgesehen, daß die Elektroden innerhalb einer Meßsonde voneinander isoliert angeordnet sind. Die Elektrolytschicht wird dann an der Unterseite bzw. Stirnseite der Meßsonde angebracht. Vorzugsweise ist die Meßsonde aus Glas oder aber einer gießfähigen Substanz, wie beispielsweise Araldit oder dgl. Entsprechend kann dann diese Substanz bereits als Isolator zwischen den Elektroden dienen.

Durch den dreieckförmigen Spannungs-/Zeitverlauf bei der zyklischen Voltammetrie an der Arbeitselektrode sind außer der qualitativen und quantitativen Information zusätzlich die Kontrolle der Oberfläche des Sensorelementes bzw. der Meßsonde und die Selbstkalibrierung des Systems möglich.

In Weiterbildung ist vorgesehen, daß unterhalb des Sensorelementes bzw. der Meßsonde eine Durchflußsonde anordbar ist. Auf diese Weise können einfach gasförmige Substanzen kontinuierlich untersucht werden. Beispielsweise ist die Durchflußzelle derart ausgebildet, daß sie auf das Sensorelement aufschraubbar ist. Die nachzuweisenden Substanzen können so selektiv und zuverlässig gemessen werden. Weiterbildungen sehen eine Dichtung zwischen Meßsonde und Durchflußzelle vor. Um das Gas an der Meßsonde vorbeizufördern, ist vorzugsweise eine Pumpeinrichtung vorgesehen. Eine Membran wie beim Stand der Technik ist bei der erfindungsgemäßen Vorrichtung zur Bestimmung gasförmiger Substanzen nicht mehr notwendig.

In weiterer bevorzugter Ausgestaltung ist vorgesehen, daß das Sensorelement bzw. die Meßsonde mit einer Regelungs- und Auswerteelektronik verbunden ist. Auf diese Weise können dann die bei angelegten Spannungswerten gemessenen Stromwerte sowie die Spannungswerte und Differenzen der Stromwerte ausgewertet und anschließend die entsprechenden Konzentrationen der nachzuweisenden Stoffe angezeigt werden. Bevorzugt ist eine Ausgabeeinheit zur Konzentrationsanzeige vorgesehen, so daß direkt beim Messen diese abgelesen werden kann. Es ist auch möglich, die Werte nicht nur optisch, sondern auch akustisch auszugeben, wobei beispielsweise eine Alarmeinheit zur Ausgabe eines Alarmsignals bei Überschreiten eines vorgegebenen Konzentrationswertes vorgesehen sein kann, wobei die akustischen Signale je nach Konzentration vorzugsweise unterschiedlich sind.

Mittels des erfindungsgemäßen Verfahrens und der erfindungsgemäßen Vorrichtung können so elektrochemisch umzusetzende Substanzen auch mit sehr kleinen Dampfdrücken qualitativ und quantitativ nachgewiesen werden. Durch den Einsatz unterschiedlicher Elektrodenmaterialien und Elektrolyte in der Meßsonde bzw. dem Sensorelement können verschiedene Substanzen selektiv detektiert werden. Aufgrund der Empfindlichkeit und Selektivität des so gegebenen Meßsystems ergeben sich Anwendungen in den unterschiedlichsten Bereichen, beispielsweise dem Umweltund Personenschutz, in dem belastende chemische Substanzen in geringen Konzentrationen nachzuweisen sind. Beispielsweise gelingt so der Nachweis von TNT aus der Gasphase. Auch können das erfindungsgemäße Verfahren und die erfindungsgemäße Vorrichtung neben beispielsweise der Suche von Minen, welche TNT enthalten, auch zur Sprengstoffdetektion bzw. bei der Sanierung kontaminierter Flächen zuverlässig und schnell eingesetzt werden. Ferner sind eine Reihe anderer Substanzen, z.B. Kampfstoffe wie Sarin, Clark I, Lewisit, Soman, Tabun sowie Lost etc., Drogen und Substanzen mit höheren Dampfdrücken, nachweisbar.

Weitere Vorteile und Merkmale der Erfindung ergeben sich aus den Ansprüchen und aus der nachfolgenden Beschreibung, in der ein Ausführungsbeispiel unter Bezugnahme auf die Zeichnung im einzelnen erläutert ist. Dabei zeigt:
- Fig. 1: eine schematische Darstellung einer Meßsonde der erfindungsgemäßen Vorrichtung im Längsschnitt;
- Fig. 2: die Anordnung einer erfindungsgemäßen Meßsonde oberhalb einer Durchflußzelle für Gase;
- Fig. 3a-c: Querschnitte durch erfindungsgemäße Meßsonden unterschiedlicher Ausführung;
- Fig. 4: eine Strom-Spannungskurve einer Trinitrotoluol (TNT) -Probe mit sechs Durchlaufzyklen;
- Fig. 5: die gegenüber der Meßzeit ausgetragenen kathodischen Strommaxima aus Fig. 4 als Funktion der Zyklusnummer;
- Fig. 6: die Stromkurve der beim kathodischen Strommaximum in Fig. 4 bei konstantem Spannungswert bestimmten Stromstärke;
- Fig. 7: eine Strom-Spannungskurve für Trinitrotoluol (TNT) mit Hexan-1-Sulfonat in Schwefelsäure als Elektrolyt und
- Fig. 8-10: Strom-Spannungskurven für drei unterschiedliche Kampfstoffe (Clark I, Sarin, Lewisit I) an einer Gold-Elektrode.

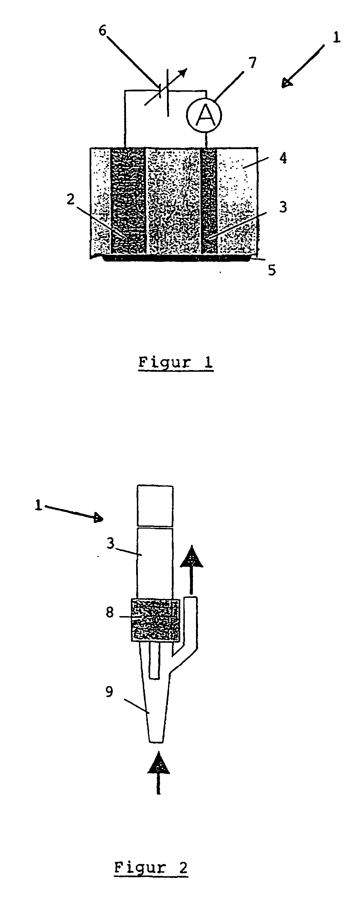

Ein in Fig. 1 dargestelltes Sensorelement bzw. eine Meßsonde 1 weist im dargestellten Ausführungsbeispiel eine Gegen- und Bezugselektrode 2 sowie eine Arbeitselektrode 3 auf. Die Arbeitselektrode 3 besitzt einen geringeren Durchmesser als die Gegen- und Bezugselektrode 2 und ist von dieser durch einen beide sowie den Zwischenraum umgebenden Isolator 4 getrennt und isoliert. An der Unterseite bzw. Stirnfläche der Meßsonde 1 ist ein Elektrolytfilm 5 aufgebracht. Dessen Kontaktfläche mit der Gegen- und Bezugselektrode 2 ist aufgrund von deren Durchmesser bedeutend größer als diejenige mit der Arbeitselektrode 3. Auf diese Weise ist die Gegen- und Bezugselektrode 2 im wesentlichen unpolarisierbar, so daß man bei dieser Konstellation mit zwei Elektroden in der Meßsonde 1 auskommen kann. Die Gegenelektrode 2 übernimmt so also die Funktion der Bezugs- oder Referenzelektrode. Die Gegen- und Bezugselektrode 2 sowie die Arbeitselektrode 3 sind mit einer gemeinsamen Spannungsquelle 6 verbunden, deren Spannung variabel regelbar ist. Des weiteren ist ein Amperometer 7 zur Messung der Stromstärke vorgesehen.

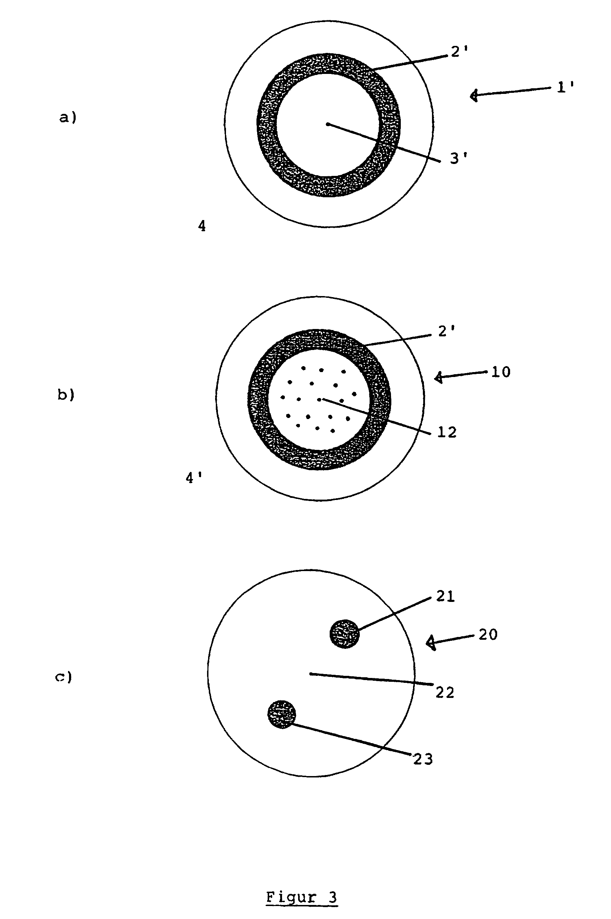

Bei dem in Fig. 2 dargestellten Ausführungsbeispiel ist das Sensorelement 1 mit seiner Arbeitselektrode 3 oberhalb einer Durchfluß- bzw. Meßzelle 9 angeordnet, durch die mittels einer nicht dargestellten Pumpe entsprechend der durch die Pfeile dargestellten Richtung ein die nachzuweisende Substanz enthaltendes Gas gepumpt wird. Zwischen der Meßzelle 9 und der Elektrode 3 ist dabei noch eine Dichtung 8 vorgesehen.

Bei dem in den Fig. 3a-c dargestellten Ausführungsbeispiel sind unterschiedliche Anordnungen und Ausführungen von Gegen-, Bezugs- und Arbeitselektrode gezeigt. Bei der in Fig. 3a dargestellten Ausführungsform ist die Gegenelektrode 2', die gleichzeitig auch als Bezugselektrode dient, ringförmig ausgebildet und umgibt die stiftförmige Arbeitselektrode 3' vollständig. Bei der in Fig. 3b dargestellten Ausführungsform sind innerhalb der ringförmigen Gegen-/Bezugselektrode 2' mehrere Arbeitselektroden 12 angeordnet. Bei der in Fig. 3c dargestellten Ausführungsform ist die Arbeitselektrode 22 zwischen der Gegenelektrode 23 und der separaten Bezugselektrode 21 innerhalb des Isolators 4' der Meßsonde 20 angeordnet. Ein derartiger Isolator 4' ist selbstverständlich auch bei der Meßsonde 10 in Fig. 3b sowie der Meßsonde 1' in Fig. 3a vorgesehen.

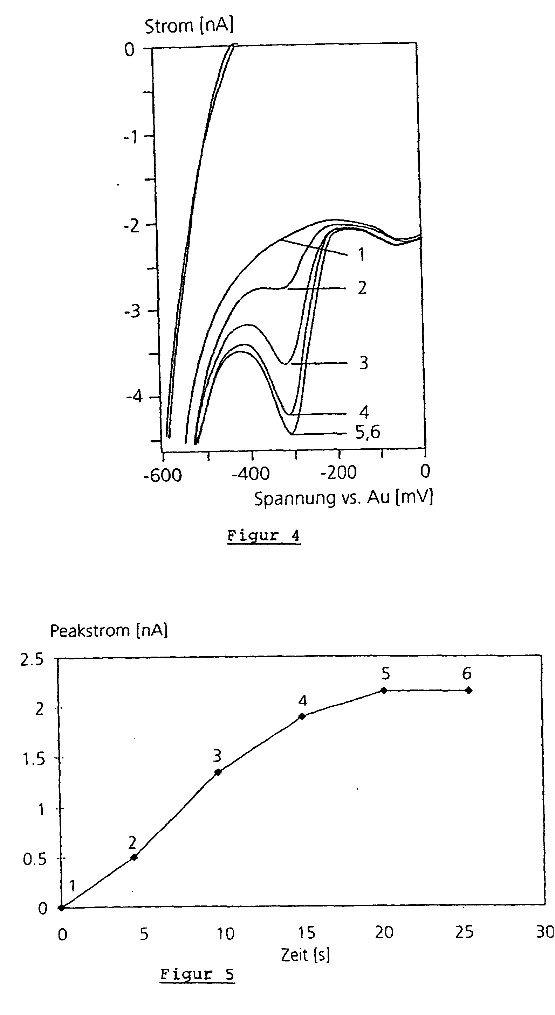

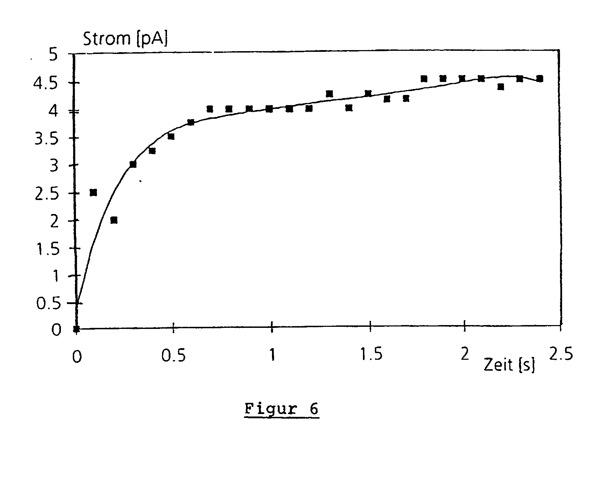

In Fig. 4 ist die Strom-Spannungskurve für eine TNT-Probe mit sechs Zyklusdurchläufen im Bereich des kathodischen Strommaximums bei -300 mV dargestellt. Wie die sechs unterschiedlichen Meßkurven zeigen, ist zu Anfang des ersten Meßzyklus 1 kein typischer Strompeak erkennbar. Dieser bildet sich erst beim zweiten Meßzyklus leicht aus, wie der Kurve 2 in Fig. 4 zu entnehmen ist. In diesem Potentialbereich bei -300 mV wird dann das in der Probe enthaltende TNT reduziert. In den nachfolgenden weiteren Meßzyklen 3 bis 6 prägt sich dieses kathodische Strommaximum immer weiter aus, wobei die beiden letzten Kurven 5 und 6 identisch sind. Diese Entwicklung des kathodischen Strommaximums ist auch der Fig. 5 zu entnehmen. Durch den Verlauf dieser Peakströme bei -300 mV läßt sich dann feststellen, daß ein kathodischer Strompeak vorliegt. Entsprechend wird nun der Potentialdurchlauf bei -300 mV angehalten und nachfolgend bei festem Spannungswert die Stromhöhe, wie in Fig. 6 dargestellt, in Abhängigkeit von der Meßzeit ermittelt. Wie Fig. 6 dabei zu entnehmen ist, tritt unter diesen potentiostatischen Bedingungen bereits nach einer Ansprechzeit von 0,5 sec ein nahezu konstanter Stromwert auf. Mittels dieses Stromwertes kann dann die Konzentration des TNT ermittelt und angegeben werden, die Stromstärke sich also kaum noch ändert.

Durch die Dicke der Elektrolytschicht kann Einfluß auf die Nachweiszeit genommen werden, so daß beispielsweise bei einem dicken Film ein konstanter Stromwert bei konstantem Potential lediglich erst nach 40 sec auftritt.

Durch die Kombination einer potentiodynamischen und potentiostatischen Messung wird zuverlässig die nachzuweisende Substanz detektierbar. Es werden bei einer Messung so lange Potentialdurchläufe vorgegeben, bis ein für die jeweilige nachzuweisende Substanz typischer Strompeak auftritt. An dieser Stelle wird dann, wie den Fig. 4 bis 6 zu entnehmen ist, der Potentialdurchlauf gestoppt und anschließend rein potentiostatisch die Veränderung des Stromes gegenüber der Meßzeit registriert.

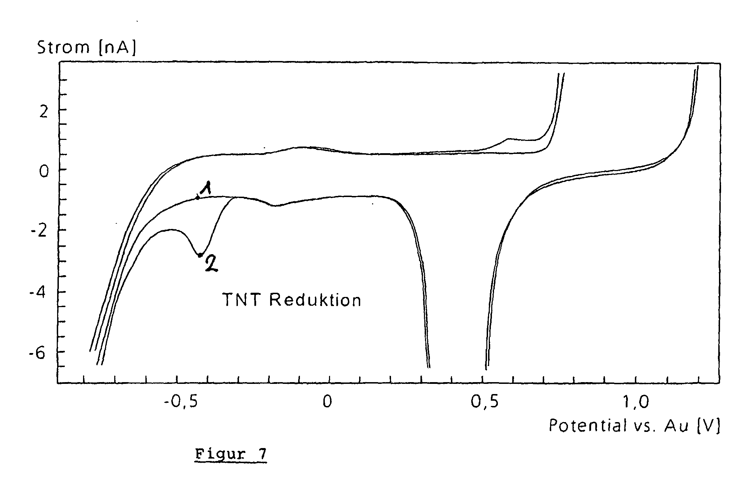

Bei einem konkreten Ausführungsbeispiel des erfindungsgemäßen Verfahrens wurde bei 20 °C die Strom-Spannungskurve für TNT, welches bei 81 °C einen Dampfdruck von 0,057 mbar aufweist, aufgenommen. Als Elektrolyt wurde Hexan-1-Sulfonat in Schwefelsäure verwendet, und bei der Arbeitselektrode handelte es sich um eine Goldelektrode. Das Potential bzw. die Spannung der Arbeitselektrode wurde in bezug zum Potential der Bezugselektrode dreieckförmig mit der Zeit variiert, um ein zyklisches Voltammogramm zu erhalten. Die Strom-Spannungs-Kurve für dieses konkrete Beispiel ist in Fig. 7 dargestellt.

Der Potentialbereich bei Aufnahme dieser Kurve liegt zwischen -600 mV und -250 mV. In diesem Bereich wird der Verlauf der Kurve auf die Ausbildung eines kathodischen Strompeaks (negatives Vorzeichen) ausgewertet. Wie die beiden unterschiedlichen Meßkurven 1 und 2 in Fig. 7 zeigen, ist dabei zu Anfang beim ersten Meßzyklus 1 kein typischer Strompeak erkennbar. Dieser bildet sich erst nach mehreren durchlaufenen Meßzyklen, wie durch die Kurve 2 dargestellt, aus. In diesem Potentialbereich wird in typischer Weise TNT reduziert. Der zugehörige Potentialwert liefert dabei die qualitative Information über das Vorliegen von TNT und die Stromhöhe eine quantitative Information über die Konzentration. Um nun festzustellen, ob ein solcher kathodischer Strompeak vorliegt, werden die Differenzen der Stromwerte der aufeinanderfolgenden Meßzyklen bestimmt und können optisch oder akustisch ausgegeben werden. Tritt nun, wie Fig. 7 zu entnehmen ist, ein kathodischer Strompeak im Potentialbereich auf, so wird der Potentialdurchlauf an diesem Potential angehalten und im weiteren bei konstantem Potential bzw. konstanter Spannung die Stromhöhe gemessen, wie dies in Fig. 6 für eine andere Probe dargestellt ist.

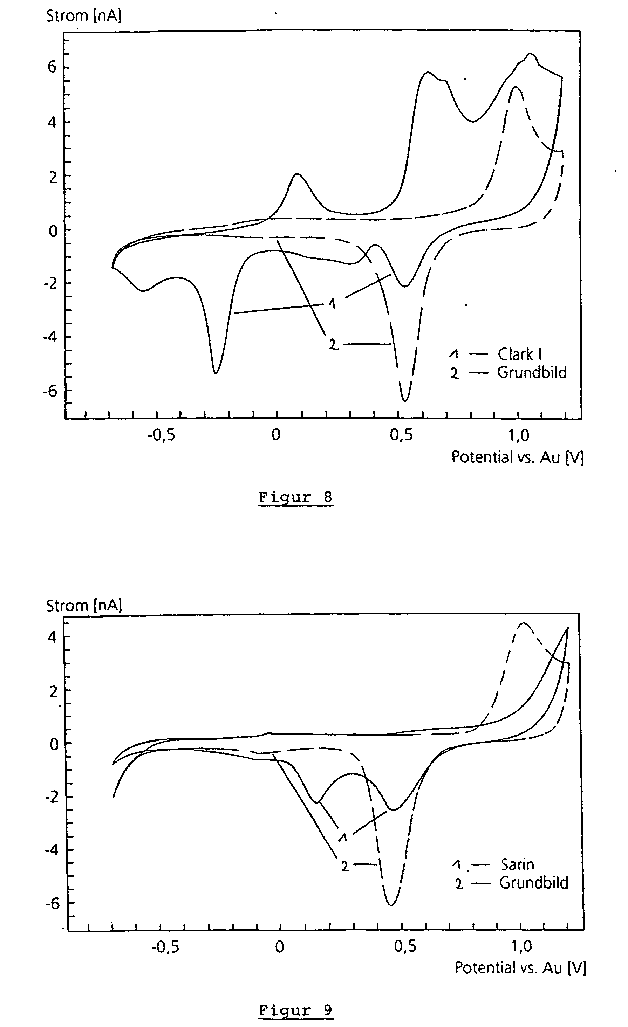

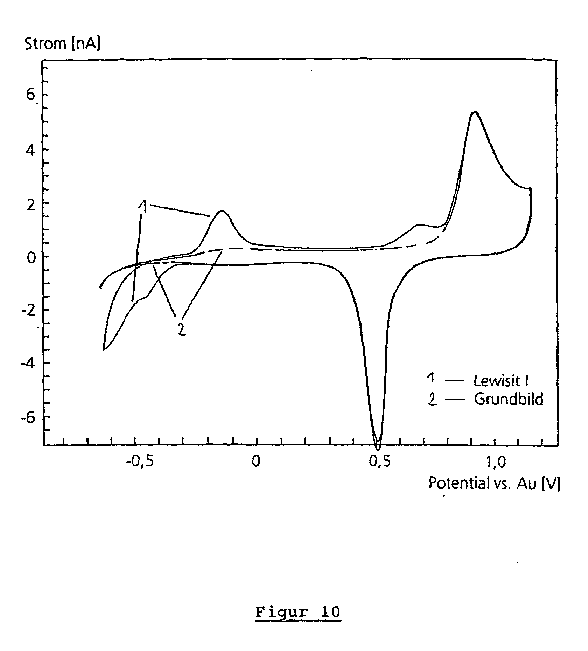

In den Fig. 8 bis 10 sind drei weitere Strom-Spannungskurven für unterschiedliche Kampfstoffe, nämlich Clark I (Fig. 8), Sarin (Fig. 9) und Lewisit I (Fig. 10) dargestellt. Als Arbeitselektrode wurde eine Goldelektrode verwendet. Das Potential bzw. die Spannung der Arbeitselektrode wurde dann ebenfalls in bezug zum Potential der Bezugselektrode dreieckförmig mit der Zeit variiert, um ein zyklisches Voltammogramm zu erhalten.

Der Potentialbereich bei Aufnahme dieser Strom-Spannungskurven liegt zwischen -700 mV und 1200 mV. In diesem Bereich wird jeweils der Verlauf der Kurve auf die Ausbildung eines kathodischen Strompeaks (negatives Vorzeichen) ausgewertet. Wie dabei die Grundbilder 2 zu Beginn einer jeden Messung in den Fig. 8-10 zeigen, ist diesen ersten Strom-Spannungskurven 2 kein substanztypischer Strompeak entnehmbar. Dieser bildet sich erst nach mehreren durchlaufenen Meßzyklen aus, wie durch die jeweilige Strom-Spannungskurve 1 in den Fig. 8-10 dargestellt ist.

Handelt es sich um den Kampfstoff Clark I, so weist die Strom-Spannungskurve 1 nach mehreren Meßzyklen einen substanzspezifischen kathodischen Strompeak bei etwa -250 mV auf. Handelt es sich um Sarin (Fig. 9), so zeigt die Strom-Spannungskurve 1 einen substanzspezifischen kathodischen Strompeak bei etwa 150 mV. Die Strom-Spannungskurve zur Bestimmung von Lewisit I (Fig. 10) weist einen kathodischen Strompeak im Bereich von etwa -600 mV auf. In diesen genannten Potentialbereichen werden in typischer Weise diese drei chemischen Kampfstoffe reduziert. Der zugehörige Potentialwert liefert dabei die qualitative Information über das Vorliegen dieser drei Kampfstoffe, während durch die Stromhöhe eine quantitative Information über die Konzentration erfolgt. Die weitere Vorgehensweise ist damit gleich derjenigen, wie sie schon unter Bezug auf die Fig. 4 bis 7 beschrieben wurde.

Wie die Fig. 8 und 10 weiterhin zeigen, weisen die Strom-Spannungskurven für Clark I sowie Lewisit I auch anodische Strommaxima im Bereich von 100 mV für Clark I und im Bereich von -150 mV für Lewisit I auf. Auch derartige substanzspezifische anodische Strommaxima, die für eine Oxidation der zu bestimmenden Substanz stehen, können ggf. zum Nachweis herangezogen werden.

## Patentansprüche

1. Detektions-Verfahren zur Detektion schwerflüchtiger Substanzen aus der Gruppe von Nitrotoluol, Trinitrotoluol, Dinitrotoluol oder Derivaten von Nitrotoluolen sowie von chemischen Kampfstoffen wie Clark, Lewisit, Lost, Sarin, Soman, Tabun oder dgl. in Böden, Flüssigkeiten und Gasen, wobei eine Elektrodenanordnung, die zumindest eine Arbeitselektrode und eine Gegenelektrode aufweist, mit den zu prüfenden Substanzen über eine über den Elektroden befindliche, in Form eines dickflüssigen oder gelartigen Films aufgebrachte und an der Stirnseite des Sensorelements haftende, dünne Elektrolyt-Schicht in Kontakt gebracht und an die Arbeitselektrode eine Spannung angelegt wird, deren Wert bei im wesentlichen übereinstimmendem Anfangs- und Endwert innerhalb eines vorgegebenen Zeitraumes zumindest einmal erhöht und verringert wird, wobei die Stromstärke während dieses zumindest einen Messzyklus in Abhängigkeit von der angelegten Spannung bestimmt wird,
- und mehrere Messzyklen durchlaufen werden bis ein kathodisches Strommaximum auftritt,
- der zugehörige Spannungswert ermittelt und konstant gehalten wird und
- die Stromstärke potentiostatisch über die Zeit bestimmt wird,
bis ein nahezu konstanter Stromwert die Konzentration der detektierten Substanz angibt.

2. Verfahren nach Anspruch 1, **dadurch gekennzeichnet, daß** bei einem Meßzyklus maximal der Potentialbereich zwischen Wasserstoff- und Sauerstoffentwicklung durchlaufen wird.

3. Verfahren nach Anspruch 1 oder 2, **dadurch gekennzeichnet, daß** die ermittelten Werte optisch ausgegeben werden.

4. Verfahren nach Anspruch 1 oder 2, **dadurch gekennzeichnet, daß** die ermittelten Werte akustisch ausgegeben werden.

5. Verfahren nach einem der Ansprüche 1 bis 4, **dadurch gekennzeichnet, daß** die Potentialdifferenz der Spannungswerte/Potentiale zwischen einem eine Sauerstoffreduktion anzeigenden anodischen Strommaximum und dem kathodischen Strommaximum bestimmt wird.

6. Verfahren nach einem der Ansprüche 1 bis 5, **dadurch gekennzeichnet, daß** eine zu untersuchende Substanz am Sensorelement vorbeigeführt wird.

7. Verfahren nach einem der Ansprüche 1 bis 6, **dadurch gekennzeichnet, daß** eine gasförmige Substanz durch Pumpen durch eine Durchflußzelle am Sensorelement vorbeigeführt wird.

8. Detektions-Vorrichtung zur Detektion schwerflüchtiger Substanzen, aus der Gruppe von Nitrotoluol, Trinitrotoluol, Dinitrotoluol oder Derivaten von Nitrotoluolen sowie von chemischen Kampfstoffen wie Clark, Lewisit, Lost, Sarin, Soman, Tabun oder dgl. in Böden, Flüssigkeiten und Gasen, nach dem Verfahren gemäß einem der vorangehenden Ansprüche, mit einem Sensorelement aus zumindest einer Arbeits- und einer Gegenelektrode, die mit einer regelbaren Spannungsquelle verbunden sind und mit einem Elektrolyten in Kontakt stehen, **dadurch gekennzeichnet,**
- **dass** auf der mit der zu untersuchenden Substanz in Kontakt tretenden Stirnfläche des Sensorelements (1), in der die Elektroden münden, eine dünne Schicht eines Elektrolyten in Form eines dickflüssigen oder gelartigen Elektrolytfilms aufgebracht ist, der an der Stirnseite des Sensorelements haftet, und
- **dass** die Vorrichtung Einrichtungen aufweist
- zum Durchlauf mehrerer Messzyklen bis zum Auftreten eines kathodischen Strommaximums,
- zur Ermittlung eines zugehörigen für eine zu findende Substanz charakteristischen Spannungswerts zur Bestimmung derselben,
- zum Konstanthalten der Spannung beim kathodischen Strommaximum und
- zur potentiostatischen Bestimmung der Stromstärke über die Zeit
- bis zum Auftreten eines nahezu konstanten, die Konzentration der aufgefundenen Substanz bestimmenden Stromwerts.

9. Vorrichtung nach einem der Ansprüche 8 bis 10, **dadurch gekennzeichnet, daß** der Elektrolyt aus organischen Verbindungen besteht.

10. Vorrichtung nach einem der Ansprüche 8 bis 10, **dadurch gekennzeichnet, daß** der Elektrolyt aus anorganischen Verbindungen besteht.

11. Vorrichtung nach einem der Ansprüche 8 bis 10, **dadurch gekennzeichnet, daß** der Elektrolyt aus einer Kombination organischer und anorganischer Verbindungen besteht.

12. Vorrichtung nach einem der Ansprüche 8 bis 13, **dadurch gekennzeichnet, daß** zumindest die Arbeits- und/oder Gegenelektrode (2, 3, 2', 3', 12, 22, 23) Mikroelektroden sind.

13. Vorrichtung nach einem der Ansprüche 8 bis 14, **dadurch gekennzeichnet, daß** die Arbeitselektrode (3') im wesentlichen ringförmig von der Gegenelektrode (2') umschlossen ist.

14. Vorrichtung nach einem der Ansprüche 8 bis 15, **gekennzeichnet durch** mehrere Arbeitselektroden (12).

15. Vorrichtung nach einem der Ansprüche 8 bis 16, **gekennzeichnet durch** eine zusätzlich vorgesehene Bezugselektrode (21).

16. Vorrichtung nach einem der Ansprüche 8 bis 17, **dadurch gekennzeichnet, daß** die Elektroden (2, 3, 2', 3', 12, 21, 22, 23) innerhalb einer Meßsonde (1, 1', 10, 20) voneinander isoliert angeordnet sind.

17. Vorrichtung nach Anspruch 18, **dadurch gekennzeichnet, daß** die Meßsonde aus Glas ist.

18. Vorrichtung nach Anspruch 18, **dadurch gekennzeichnet, daß** die Meßsonde aus einer gießfähigen Substanz ist.

19. Vorrichtung nach Anspruch 20, **dadurch gekennzeichnet, daß** die gießfähige Substanz Araldit oder dgl. ist.

20. Vorrichtung nach einem der Ansprüche 8 bis 21, **dadurch gekennzeichnet, daß** unterhalb des Sensorelementes bzw. der Meßsonde (1) eine Durchflußzelle bzw. Meßzelle (9) anordbar ist.

21. Vorrichtung nach Anspruch 22, **dadurch gekennzeichnet, daß** die Durchfluß- bzw. Meßzelle (9) auf dem Sensorelement bzw. der Meßsonde (1) zu befestigen ist.

22. Vorrichtung nach Anspruch 23, **dadurch gekennzeichnet, daß** die Durchfluß- bzw. Meßzelle (9) und das Sensorelement bzw. die Meßsonde (1) durch eine Dichtung (8) voneinander getrennt sind.

23. Vorrichtung nach einem der Ansprüche 22 bis 24, **gekennzeichnet durch** eine Pumpeinrichtung zum Pumpen des zu untersuchenden Gases **durch** die Durchfluß- bzw. Meßzelle (9).

24. Vorrichtung nach einem der Ansprüche 8 bis 25, **dadurch gekennzeichnet, daß** das Sensorelement bzw. die Meßsonde (1) eine Regelungs- und Auswerteelektronik aufweist.

25. Vorrichtung nach einem der Ansprüche 8 bis 26, **dadurch gekennzeichnet, daß** die Vorrichtung eine Ausgabeeinheit zur Konzentrationsanzeige aufweist.

## Claims

1. Detection method for detecting low-volatility substances from the group of nitrotoluene, trinitrotoluene, dinitrotoluene or derivatives of nitrotoluenes and of chemical warfare agents, such as clark, lewisite, mustard gas, sarin, soman, tabun or the like, in soils, liquids and gases, an electrode arrangement, which has at least one working electrode and one counterelectrode, being brought into contact with the substances to be tested via a thin electrolyte layer which is situated over the electrodes, is applied in the form of a viscous or gel-like film and adheres to the end-side of the sensor element, and a voltage being applied to the working electrode, the value of which is increased and reduced at least once within a prescribed period of time with a substantially corresponding initial and final value, the current strength during this at least one measuring cycle being determined dependent upon the applied voltage,
- and a plurality of measuring cycles being passed through until a cathodic current maximum occurs,
- the associated voltage value being determined and maintained constant and
- the current strength being determined potentiostatically over the time,
until a virtually constant current value indicates the concentration of the detected substance.

2. Method according to claim 1, **characterised in that**, during one measuring cycle, at most the potential range between hydrogen and oxygen evolution is passed through.

3. Method according to claim 1 or 2, **characterised in that** the determined values are output optically.

4. Method according to claim 1 or 2, **characterised in that** the determined values are output acoustically.

5. Method according to one of the claims 1 to 4, **characterised in that** the potential difference of the voltage values/potentials between an anodic current maximum, which indicates an oxygen reduction, and the cathodic current maximum is determined.

6. Method according to one of the claims 1 to 5, **characterised in that** a substance to be tested is conducted past the sensor element.

7. Method according to one of the claims 1 to 6, **characterised in that** a gaseous substance is conducted past the sensor element by pumping through a flow-through cell.

8. Detection device for detecting low-volatility substances from the group of nitrotoluene, trinitrotoluene, dinitrotoluene or derivatives of nitrotoluenes and of chemical warfare agents, such as clark, lewisite, mustard gas, sarin, soman, tabun or the like, in soils, liquids and gases, according to the method according to one of the preceding claims, having a sensor element comprising at least one working and one counterelectrode which are connected to a controllable voltage source and are in contact with an electrolyte, **characterised in that**
- a thin layer of an electrolyte is applied, in the form of a viscous or gel-like electrolyte film which adheres to the end-side of the sensor element, upon the end-face of the sensor element (1), which face comes into contact with the substance to be tested and in which the electrodes open out and
- **in that** the device has apparatuses,
- for passing through a plurality of measuring cycles until a cathodic current maximum occurs,
- for determining an associated voltage value characteristic for a substance to be found in order to determine said voltage value,
- for maintaining the voltage constant during the cathodic current maximum and
- for potentiostatic determination of the current strength over the time
- until a virtually constant current value occurs which determines the concentration of the detected substance.

9. Device according to claim 8, **characterised in that** the electrolyte comprises organic compounds.

10. Device according to claim 8, **characterised in that** the electrolyte comprises inorganic compounds.

11. Device according to claim 8, **characterised in that** the electrolyte comprises a combination of organic and inorganic compounds.

12. Device according to one of the claims 8 to 11, **characterised in that** at least the working and/or counterelectrode (2, 3, 2', 3', 12, 22, 23) are microelectrodes.

13. Device according to one of the claims 8 to 12, **characterised in that** the working electrode (3') is substantially annularly enclosed by the counterelectrode (2').

14. Device according to one of the claims 8 to 13, **characterised by** a plurality of working electrodes (12).

15. Device according to one of the claims 8 to 14, **characterised by** an additionally provided reference electrode (21).

16. Device according to one of the claims 8 to 15, **characterised in that** the electrodes (2, 3, 2', 3', 12, 21, 22, 23) are disposed insulated from each other within a sensing probe (1, 1', 10, 20).

17. Device according to claim 16, **characterised in that** the sensing probe is made of glass.

18. Device according to claim 16, **characterised in that** the sensing probe is made of a castable substance.

19. Device according to claim 18, **characterised in that** the castable substance is Araldite or the like.

20. Device according to one of the claims 8 to 19, **characterised in that** a flow-through cell or measuring cell (9) can be disposed under the sensor element or the sensing probe (1).

21. Device according to claim 20, **characterised in that** the flow-through or measuring cell (9) is able to be mounted on the sensor element or the sensing probe (1).

22. Device according to claim 21, **characterised in that** the flow-through or measuring cell (9) and the sensor element or the sensing probe (1) are separated from each other by means of a seal (8).

23. Device according to one of the claims 20 to 22, **characterised by** a pumping apparatus for pumping the gas to be tested through the flow-through or measuring cell (9).

24. Device according to one of the claims 8 to 23, **characterised in that** the sensor element or the sensing probe (1) has a control and evaluation electronics unit.

25. Device according to one of the claims 8 to 24, **characterised in that** the device has an output unit for displaying the concentration.

## Revendications

1. Procédé de détection de substances peu volatiles du groupe des nitrotoluènes, des trinitrotoluènes, des dinitrotoluènes ou des dérivés de nitrotoluènes, ainsi que des agents de combat chimiques tels que le clark, la lewisite, l'ypérite, le sarin, le soman, le tabun ou similaires, dans des sols, des liquides et des gaz,
selon lequel
- un ensemble d'électrodes comprenant au moins une électrode de travail et une contre-électrode est mis en contact avec les substances à détecter par l'intermédiaire d'une couche d'électrolyte mince située au-dessus des électrodes, appliquée sous la forme d'un film visqueux ou ayant la consistance d'un gel et adhérant à la face frontale de l'élément capteur,
- on applique sur l'électrode de travail une tension dont la valeur, pour des valeurs initiale et finale pour l'essentiel concordantes, est au moins une fois augmentée et réduite à l'intérieur d'une période prédéterminée, l'intensité de courant pendant au moins un cycle de mesure étant déterminée en fonction de la tension appliquée,
- on effectue plusieurs cycles de mesure jusqu'à obtenir un maximum de courant cathodique,
- la valeur de tension associée est déterminée et maintenue constante, et
- l'intensité de courant est déterminée de façon potentiostatique sur le temps,
jusqu'à ce qu'une valeur de courant sensiblement constante indique la concentration de la substance détectée.

2. Procédé selon la revendication 1,
**caractérisé en ce que**
lors d'un cycle de mesure au maximum la plage de potentiel entre le développement d'hydrogène et d'oxygène est prise en compte.

3. Procédé selon la revendication 1 ou 2,
**caractérisé en ce que**
les valeurs détectées sont indiquées optiquement.

4. Procédé selon la revendication 1 ou 2,
**caractérisé en ce que**
les valeurs détectées sont indiquées acoustiquement.

5. Procédé selon l'une quelconque des revendications 1 à 4,
**caractérisé en ce que**
la différence de potentiel des valeurs de tension/potentiels est déterminée entre un maximum de courant anodique indicateur d'une réduction d'oxygène et le maximum de courant cathodique.

6. Procédé selon l'une quelconque des revendications 1 à 5,
**caractérisé en ce qu'**
on fait passer une substance à examiner devant l'élément capteur.

7. Procédé selon l'une quelconque des revendications 1 à 6,
**caractérisé en ce qu'**
on fait passer une substance gazeuse devant l'élément capteur par pompage à travers une cellule de passage.

8. Dispositif de détection de substances peu volatiles du groupe des nitrotoluènes, des trinitrotoluènes, des dinitrotoluènes ou des dérivés de nitrotoluènes, ainsi que des agents de combat chimiques tels que le clark, la lewisite, l'ypérite, le sarin, le soman, le tabun ou similaires, dans des sols, des liquides et des gaz, suivant le procédé selon l'une quelconque des revendications précédentes, comprenant un élément capteur avec au moins une électrode de travail et une contre-électrode reliées à une source de tension réglable et en contact avec un électrolyte,
**caractérisé en ce que**
- sur la face frontale de l'élément capteur (1) entrant en contact avec la substance à examiner, dans laquelle débouchent les électrodes, une couche mince d'un électrolyte sous la forme d'un film d'électrolyte visqueux ou ayant la consistance d'un gel est appliquée et adhère à la face frontale de l'élément capteur, et
- le dispositif présente des installations permettant :
- le passage de plusieurs cycles de mesure jusqu'à l'obtention d'un maximum de courant cathodique,
- la détection d'une valeur de tension associée caractéristique d'une substance recherchée pour déterminer celle-ci,
- le maintien d'une tension constante au maximum de courant cathodique, et
- la détermination potentiostatique de l'intensité de courant dans le temps,
- jusqu'à obtenir une valeur de courant sensiblement constante déterminant la substance trouvée.

9. Dispositif selon la revendication 8,
**caractérisé en ce que**
l'électrolyte est composé de liaisons organiques.

10. Dispositif selon la revendication 8,
**caractérisé en ce que**
l'électrolyte est composé de liaisons anorganiques.

11. Dispositif selon la revendication 8,
**caractérisé en ce que**
l'électrolyte est composé d'une combinaison de liaisons organiques et anorganiques.

12. Dispositif selon l'une quelconque des revendications 8 à 11,
**caractérisé en qu'**
au moins l'électrode de travail et/ou la contre-électrode (2, 3, 2', 3', 12, 22, 23) est une microélectrode.

13. Dispositif selon l'une quelconque des revendications 8 à 12,
**caractérisé en ce que**
l'électrode de travail (31 est entourée par la contre-électrode (21 pratiquement de façon annulaire.

14. Dispositif selon l'une quelconque des revendications 8 à 13,
**caractérisé par**
plusieurs électrodes de travail (12).

15. Dispositif selon l'une quelconque des revendications 8 à 14,
**caractérisé par**
une électrode de référence (21) supplémentaire.

16. Dispositif selon l'une quelconque des revendications 8 à 15,
**caractérisé en ce que**
les électrodes (2, 3, 2', 3', 12, 21, 22, 23) sont isolées les unes des autres à l'intérieur d'une sonde de mesure (1, 1', 10, 20).

17. Dispositif selon la revendication 16,
**caractérisé en ce que**
la sonde de mesure est en verre.

18. Dispositif selon la revendication 16,
**caractérisé en ce que**
la sonde de mesure est réalisée à partir d'une substance pouvant être coulée.

19. Dispositif selon la revendication 18,
**caractérisé en ce que**
la substance pouvant être coulée est l'Araldite ou similaire.

20. Dispositif selon l'une quelconque des revendications 8 à 19,
**caractérisé en ce qu'**
une cellule de passage ou cellule de mesure (9) peut être disposée sous l'élément capteur ou la sonde de mesure (1).

21. Dispositif selon la revendication 20,
**caractérisé en ce que**
la cellule de passage ou de mesure (9) est à fixer sur l'élément capteur ou la sonde de mesure (1).

22. Dispositif selon la revendication 21,
**caractérisé en ce que**
la cellule de passage ou de mesure (9) et l'élément capteur ou la sonde de mesure (1) sont séparés l'un de l'autre par une garniture d'étanchéité (8).

23. Dispositif selon l'une quelconque des revendications 20 à 22,
**caractérisé par**
une installation de pompage pour pomper le gaz à examiner à travers la cellule de passage ou de mesure (9).

24. Dispositif selon l'une quelconque des revendications 8 à 23,
**caractérisé en ce que**
l'élément capteur ou la sonde de mesure (1) présente une électronique de régulation et d'interprétation.

25. Dispositif selon l'une quelconque des revendications 8 à 24,
**caractérisé en ce que**
le dispositif présente une unité d'émission pour indiquer la concentration.
